# EUROPEAN PATENT APPLICATION

(11) **EP 2 679 582 A1**
(43) Date of publication of application: **01.01.2014**
(21) Application number: 12749865.7
(22) Date of filing: 23.02.2012
(51) Int. Cl.: C07D 213/22, C07D 213/38, C09K 11/06, H05B 33/14, H05B 33/22

(54) **COMPOUND CONTAINING SUBSTITUTED O-TERPHENYL STRUCTURE, AND ORGANIC ELECTROLUMINESCENT ELEMENT**

(30) Priority: 23.02.2011 JP 2011037323
(71) Applicant: Hodogaya Chemical Co., Ltd., Tokyo 104-0028 (JP); Kyushu University, National University Corporation, Fukuoka-shi, Fukuoka 812-8581 (JP)
(72) Inventor: ADACHI, Chihaya, Fukuoka-shi Fukuoka 812-8581 (JP); YASUDA, Takuma, Fukuoka-shi Fukuoka 812-8581 (JP); TOGASHI, Kazunori, Tokyo 104-0028 (JP); NOMURA, Shintaro, Fukuoka-shi Fukuoka 812-8581 (JP)
(74) Representative: Ehlich, Hendrik
(86) International application number: PCT/JP2012/001218
(87) International publication number: WO 2012/114746

(57) **Abstract**

A light-emitting-layer host material is provided as material for high-efficiency organic electroluminescent devices. The light-emitting-layer host material has a high excitation triplet level, and is capable of completely confining the triplet excitons of phosphorescent material. A high-efficiency and high-luminance organic electroluminescent device is provided by using the compound. The compound is a compound of general formula (1) having a bipyridyl group and an ortho-terphenyl structure. The organic electroluminescent device includes a pair of electrodes, and one or more organic layers sandwiched between the pair of electrodes, and uses the compound as constituent material of at least one of the organic layers.

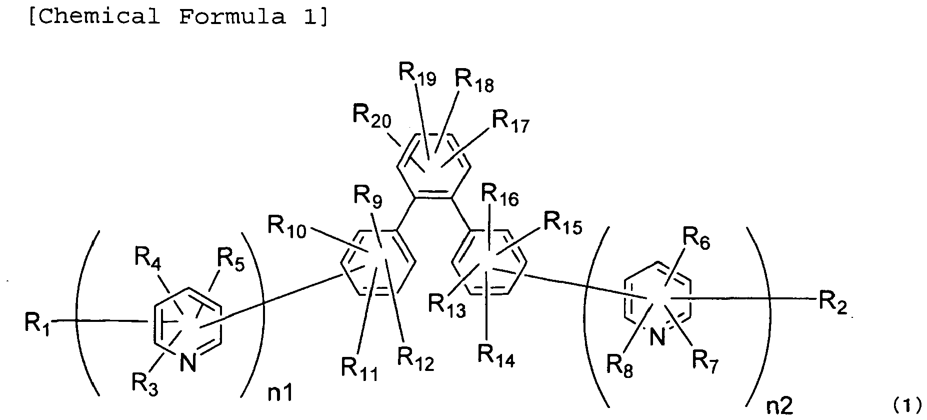

## Description

### Technical Field

The present invention relates to compounds suited for an organic electroluminescent device, a preferred self light-emitting device for various display devices, and to the device. Specifically, the invention relates to compounds having a bipyridyl group and an ortho-terphenyl structure, and to organic electroluminescent devices that use the compounds.

### Background Art

The organic electroluminescent device is a self-emitting device, and has been actively studied for their brighter, superior viewability and ability to display clearer images compared with the liquid crystal device.

In an attempt to improve the device luminous efficiency, there have been developed devices that use phosphorescent materials to generate phosphorescence, specifically that make use of the emission from the triplet excitation state. According to the excitation state theory, phosphorescent materials are expected to greatly improve luminous efficiency as much as about four times that of the conventional fluorescence.
In 1993, M.A. Baldo et al. at Princeton University realized 8% external quantum efficiency with a phosphorescent device using an iridium complex.

Because phosphorescent materials undergo concentration quenching, a charge-transporting compound, or a host compound as it is generally called, is used to support the phosphorescent materials by being doped with the phosphorescent materials. The phosphorescent materials so supported are called guest compounds. 4,4'-Di(N-carbazolyl)biphenyl (hereinafter, referred to simply as "CBP") represented by the following formula is commonly used as the host compound (refer to Non-Patent Document 1, for example).

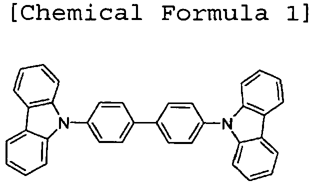

However, because of the low glass transition point (Tg) of 62°C and high crystallinity, it has been indicated that CBP lacks stability in the thin-film state. The device characteristics are thus unsatisfactory in situations where heat resistance is needed such as in emitting light of high luminance.

Advances in phosphorescent device studies have promoted further understanding of the energy transfer process between the phosphorescent material and the host compound. Studies found that the host compound needs to have a higher excitation triplet level than the phosphorescent material in order to increase luminous efficiency.

The external quantum efficiency of a phosphorescent device remains at about 6% when the blue phosphorescent material FIrpic of the formula below is doped to CBP to provide the host compound of the light emitting layer. This is considered to be due to the lower excitation triplet level of CBP, 2.57 eV, than the excitation triplet level, 2.67 eV, of FIrpic, making it difficult for the FIrpic to sufficiently confine triplet excitons. This has been demonstrated by the temperature dependence of the photoluminescence intensity of a thin film produced by the doping of CBP with FIrpic (refer to Non-Patent Document 2).

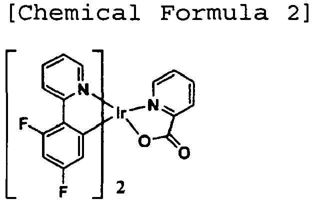

The host compound 1,3-bis(carbazol-9-yl)benzene (hereinafter, referred to simply as "mCP") of the formula below is known to have a higher excitation triplet level than CBP. However, as with the case of CBP, mCP has a low glass transition point (Tg) of 55°C and high crystallinity, and lacks stability in the thin-film state. The device characteristics are thus unsatisfactory in situations where heat resistance is needed such as in emitting light of high luminance (refer to Non-Patent Document 2).

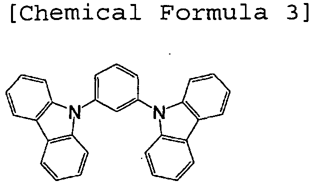

It has been found from the studies of host compounds of higher excitation triplet levels that doping an iridium complex to an electron transporting host compound or a bipolar transporting host compound can produce high luminous efficiency (refer to Non-Patent Document 3, for example).

As described above, in order to improve the luminous efficiency of a phosphorescent device in actual settings, a light-emitting-layer host compound is needed that has a high excitation triplet level, and high thin-film stability.

### Citation List

### Non-Patent Documents

Non-Patent Document 1: Appl. Phys. Let., 75, 4 (1999)
Non-Patent Document 2: Development and Evaluation Techniques for Organic EL Illumination Materials, p102-106, Science & Technology, (2010)
Non-Patent Document 3: Organic EL Display p90, Ohm Electric, Ltd. (2005)
Non-Patent Document 4: J. Org. Chem., 60, 7508 (1995)
Non-Patent Document 5: Synth. Commun., 11, 513 (1981)
Non-Patent Document 6: Organic EL Symposium, the 1st Regular presentation prints, 19 (2005)

### Summary of the Invention

### Problems that the Invention is to Solve

It is an object of the present invention to provide a light-emitting-layer host compound for use as material of a high-efficiency organic electroluminescent device, and that has a high excitation triplet level, and is capable of completely confining the triplet excitons of phosphorescent material. The invention also provides a high-efficiency and high-luminance organic electroluminescent device by using the compound. Some of the physical properties of the organic compounds to be provided by the present invention include (1) high excitation triplet level, (2) bipolar transport, and (3) stable thin-film state. Some of the physical properties of the organic electroluminescent device to be provided by the present invention include (1) high luminous efficiency, (2) high emission luminance, and (3) low actual driving voltage.

### Means for Solving the Problems

In order to achieve the foregoing object, the present inventors focused on the electron transporting ability of a bipyridyl structure, and designed and chemically synthesized compounds by using energy levels as indices. Upon confirming the energy levels of the compounds by actual measurements of work functions, novel compounds having a bipyridyl group and an ortho-terphenyl structure were found that are suitable for phosphorescent devices. The compounds were used to fabricate various test organic electroluminescent devices, and device characteristics were evaluated to complete the present invention.

1) Specifically, the present invention is a compound of general formula (1) having a bipyridyl group and an ortho-terphenyl structure.

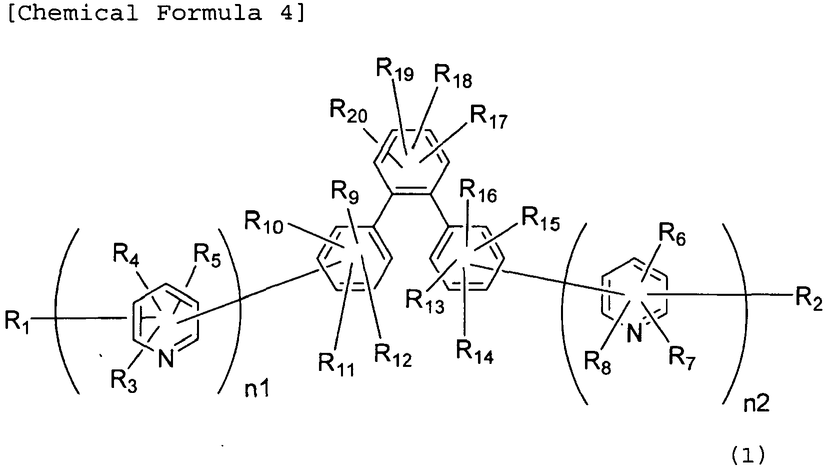

In the formula, R₁ to R₂₀ may be the same or different, and represent a hydrogen atom, a deuterium atom, a fluorine atom, a chlorine atom, cyano, trifluoromethyl, linear or branched alkyl of 1 to 6 carbon atoms that may have a substituent, a substituted or unsubstituted aromatic hydrocarbon group, a substituted or unsubstituted aromatic heterocyclic group, or a substituted or unsubstituted condensed polycyclic aromatic group. n1 and n2 may be the same or different, and represent 2 or 3. The plurality of R₃ to R₈ may be the same or different, respectively.

2) The present invention is a compound of the following general formula (1') having a bipyridyl group and an ortho-terphenyl structure.

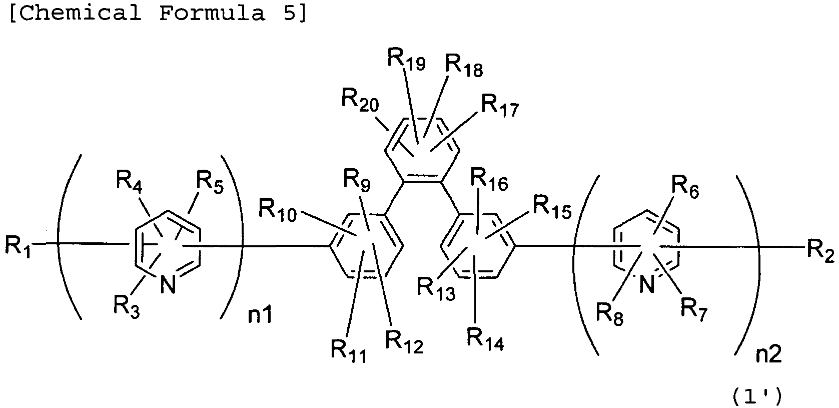

In the formula, R₁ to R₂₀ may be the same or different, and represent a hydrogen atom, a deuterium atom, a fluorine atom, a chlorine atom, cyano, trifluoromethyl, linear or branched alkyl of 1 to 6 carbon atoms that may have a substituent, a substituted or unsubstituted aromatic hydrocarbon group, a substituted or unsubstituted aromatic heterocyclic group, or a substituted or unsubstituted condensed polycyclic aromatic group. n1 and n2 may be the same or different, and represent 2 or 3. The plurality of R₃ to R₈ may be the same or different, respectively.

3) The present invention is a compound of the following general formula (1") having a bipyridyl group and an ortho-terphenyl structure.

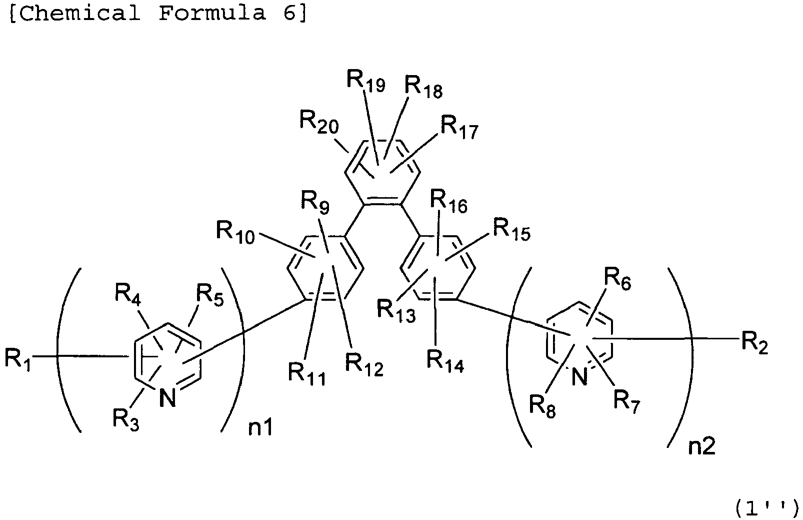

In the formula, R₁ to R₂₀ may be the same or different, and represent a hydrogen atom, a deuterium atom, a fluorine atom, a chlorine atom, cyano, trifluoromethyl, linear or branched alkyl of 1 to 6 carbon atoms that may have a substituent, a substituted or unsubstituted aromatic hydrocarbon group, a substituted or unsubstituted aromatic heterocyclic group, or a substituted or unsubstituted condensed polycyclic aromatic group. n1 and n2 may be the same or different, and represent 2 or 3. The plurality of R₃ to R₈ may be the same or different, respectively.

4) The present invention is an organic electroluminescent device that includes a pair of electrodes, and one or more organic layers sandwiched between the pair of electrodes, wherein at least one of the organic layers contains the compound of the general formula (1), (1'), or (1") having a bipyridyl group and an ortho-terphenyl structure.

5) The present invention is the organic electroluminescent device of 4), wherein the organic layer is a light emitting layer, and wherein the compound of the general formula (1), (1'), or (1") having a bipyridyl group and an ortho-terphenyl structure is used as at least one of constituent materials in the light emitting layer.

6) The present invention is the organic electroluminescent device of 4), wherein the organic electroluminescent device includes a pair of electrodes, and a phosphorescent light-emitting material-containing light emitting layer and one or more organic layers sandwiched between the pair of electrodes, and wherein the compound of the general formula (1), (1'), or (1") having a bipyridyl group and an ortho-terphenyl structure is used as at least one of constituent materials in the light emitting layer.

7) The present invention is the organic electroluminescent device of any one of 4) to 6), wherein the phosphorescent light-emitting material is a metal complex that contains iridium or platinum.

Specific examples of the "alkyl" in the "linear or branched alkyl of 1 to 6 carbon atoms that may have a substituent" represented by R₁ to R₂₀ in the general formula (1), (1'), or (1") include methyl, ethyl, n-propyl, isopropyl, n-butyl, isobutyl, tert-butyl, n-pentyl, isopentyl, neopentyl, and n-hexyl.

Specific examples of the "substituent" in the "substituted linear or branched alkyl of 1 to 6 carbon atoms" represented by R₁ to R₂₀ in the general formula (1), (1'), or (1") include a deuterium atom, a fluorine atom, a chlorine atom, cyano, trifluoromethyl, nitro, linear or branched alkyl of 1 to 6 carbon atoms, cyclopentyl, cyclohexyl, linear or branched alkoxy of 1 to 6 carbon atoms, dialkylamino substituted with linear or branched alkyl of 1 to 6 carbon atoms, phenyl, biphenylyl, terphenylyl, tetrakisphenyl, styryl, naphthyl, fluorenyl, phenanthryl, indenyl, pyrenyl, pyridyl, bipyridyl, triazyl, pyrimidyl, quinolyl, isoquinolyl, indolyl, pyridoindolyl, carbazolyl, quinoxalyl, and pyrazolyl. These substituents may be further substituted, and may be bound to each other to form a ring.

Specific examples of the "aromatic hydrocarbon group", "aromatic heterocyclic group", or "condensed polycyclic aromatic group" in the "substituted or unsubstituted aromatic hydrocarbon group", "substituted or unsubstituted aromatic heterocyclic group", or "substituted or unsubstituted condensed polycyclic aromatic group" represented by R₁ to R₂₀ in the general formula (1), (1'), or (1") include phenyl, biphenylyl, terphenylyl, tetrakisphenyl, styryl, naphthyl, anthryl, acenaphthenyl, fluorenyl, phenanthryl, indenyl, pyrenyl, pyridyl, bipyridyl, triazyl, pyrimidyl, furanyl, pyrrolyl, thienyl, quinolyl, isoquinolyl, benzofuranyl, benzothienyl, indolyl, carbazolyl, benzooxazolyl, benzothiazolyl, quinoxalyl, benzoimidazolyl, pyrazolyl, pyridoindolyl, dibenzofuranyl, dibenzothienyl, naphthyridinyl, phenanthrolinyl, and acridinyl.
Preferred as the "substituted or unsubstituted aromatic heterocyclic group" represented by R₁ to R₈ is substituted or unsubstituted pyridyl, because it can be expected to improve the electron transport characteristic.

Specific examples of the "substituent" in the "substituted aromatic hydrocarbon group", "substituted aromatic heterocyclic group", or "substituted condensed polycyclic aromatic group" represented by R₁ to R₂₀ in the general formula (1), (1'), or (1") include a deuterium atom, a fluorine atom, a chlorine atom, cyano, trifluoromethyl, nitro, linear or branched alkyl of 1 to 6 carbon atoms, cyclopentyl, cyclohexyl, linear or branched alkoxy of 1 to 6 carbon atoms, dialkylamino substituted with linear or branched alkyl of 1 to 6 carbon atoms, phenyl, biphenylyl, terphenylyl, tetrakisphenyl, styryl, naphthyl, fluorenyl, phenanthryl, indenyl, pyrenyl, pyridyl, bipyridyl, triazyl, pyrimidyl, quinolyl, isoquinolyl, indolyl, pyridoindolyl, carbazolyl, quinoxalyl, and pyrazolyl. These substituents may be further substituted.

The compounds of the general formula (1), (1'), or (1") having a bipyridyl group and an ortho-terphenyl structure are novel compounds, and have preferable energy levels as host compounds of a light emitting layer, and an excellent triplet exciton confining capability.

The compounds of the general formula (1), (1'), or (1") having a bipyridyl group and an ortho-terphenyl structure of the present invention can be used as constituent materials of the light emitting layer or hole blocking layer of an organic electroluminescent device (hereinafter, referred to simply as "organic EL device"). The compounds of the present invention, having a more desirable bipolar transport characteristic compared to conventional materials, have the effects to improve power efficiency, and lower actual driving voltage.

### Advantage of the Invention

The compounds having a bipyridyl group and an ortho-terphenyl structure of the present invention are useful as the hole blocking compounds of an organic EL device, or the host compounds of the light emitting layer of an organic EL device. The organic EL device produced by using the compounds can have high efficiency, high luminance, and low driving voltage.

### Brief Description of the Drawings

FIG. 1 is a ¹H-NMR chart of the compound of Example 1 of the present invention (Compound 2).
FIG. 2 is a ¹H-NMR chart of the compound of Example 2 of the present invention (Compound 3).
FIG. 3 is a ¹H-NMR chart of the compound of Example 3 of the present invention (Compound 10).
FIG. 4 is a ¹H-NMR chart of the compound of Example 4 of the present invention (Compound 11).
FIG. 5 is a diagram representing the configuration of the EL devices of Examples 8 to 10 and Comparative Example 1.
FIG. 6 is a diagram representing the configuration of the EL devices of Comparative Example 2.

### Mode for Carrying Out the Invention

The compounds having a bipyridyl group and an ortho-terphenyl structure of the present invention are novel compounds, and may be synthesized by using, for example, the following method. First, the dihalide of a corresponding ortho-terphenylene compound is boronated with a compound such as bis(pinacolato)diboron to synthesize a corresponding borate product (refer to Non-Patent Document 4, for example), and this corresponding borate product is reacted with a halogenobipyridine having various substituents in a cross-coupling reaction such as Suzuki coupling (refer to Non-Patent Document 5, for example) to synthesize the compound having a bipyridyl group and an ortho-terphenyl structure.
The compounds having a bipyridyl group and an ortho-terphenyl structure also can be synthesized as follows. First, a halogenobipyridine having various substituents is boronated with a compound such as bis (pinacolato) diboron, and the resulting bipyridine borate product with various substituents is then reacted with the dihalide of a corresponding ortho-terphenylene compound in a cross-coupling reaction such as Suzuki coupling.

The following presents specific examples of preferred compounds among the compounds of general formula (1), (1'), or (1") having a bipyridyl group and an ortho-terphenyl structure. The present invention, however, is not limited to these compounds.

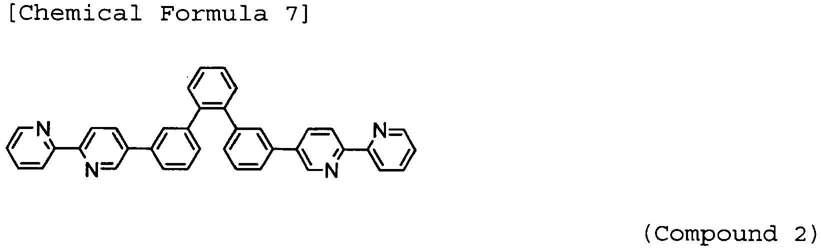

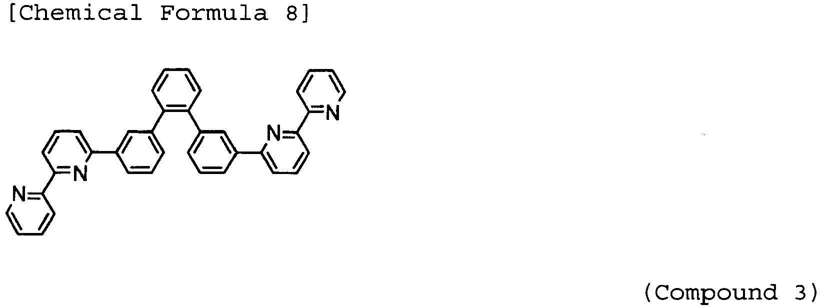

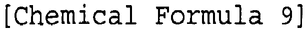

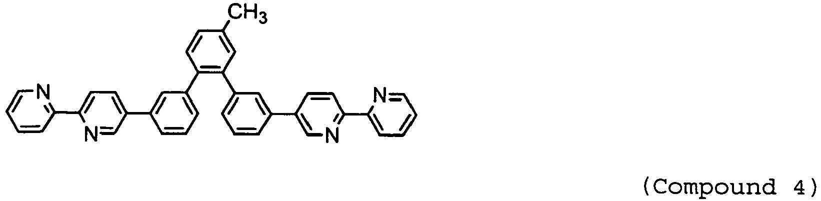

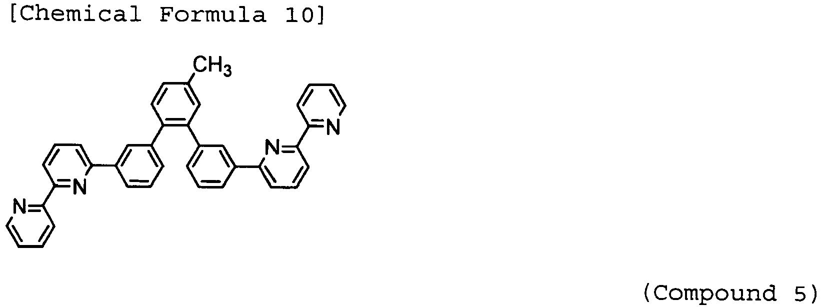

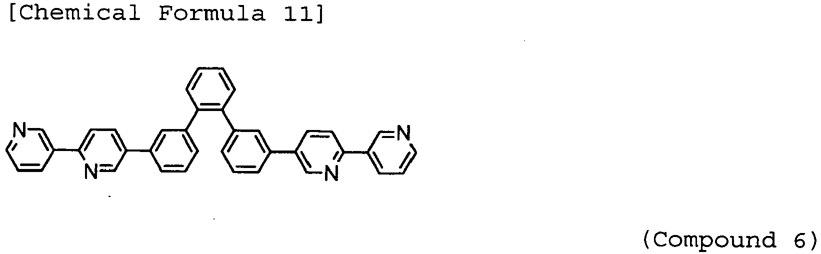

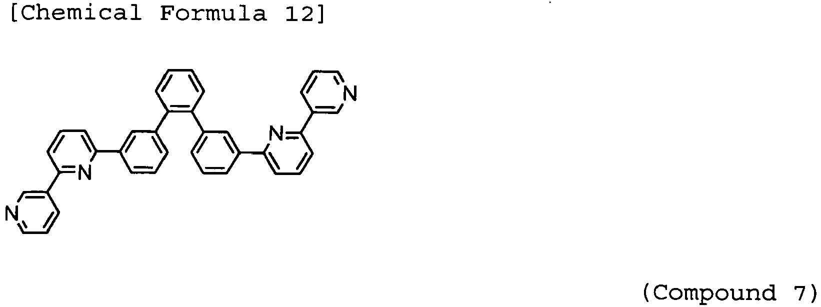

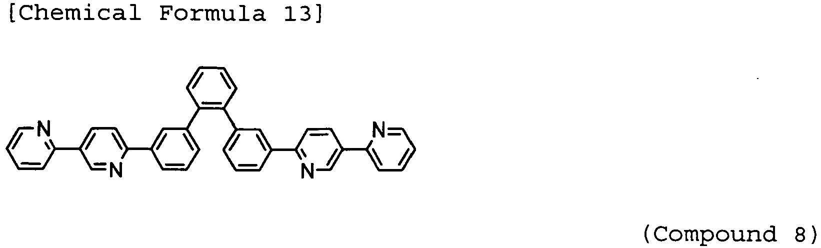

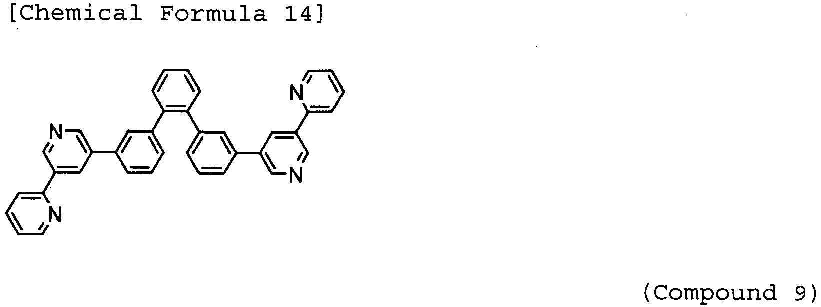

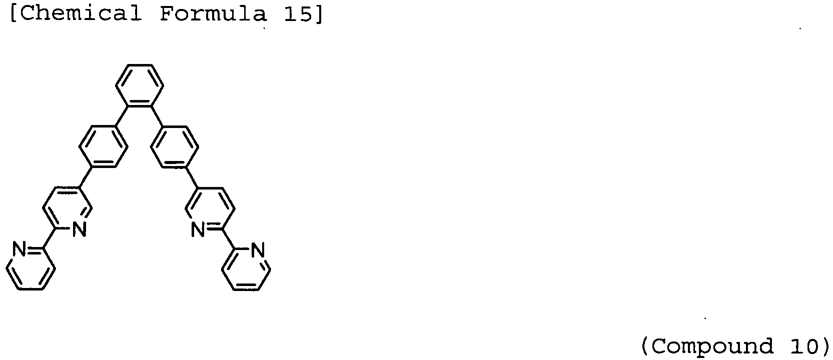

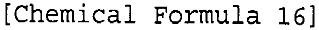

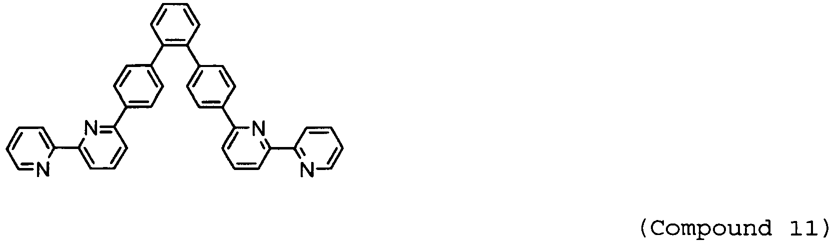

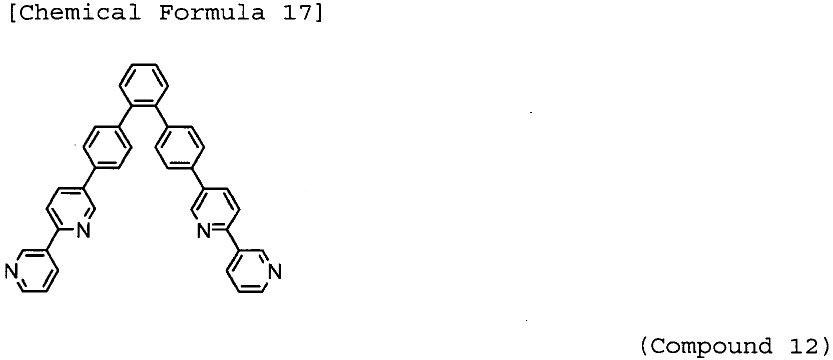

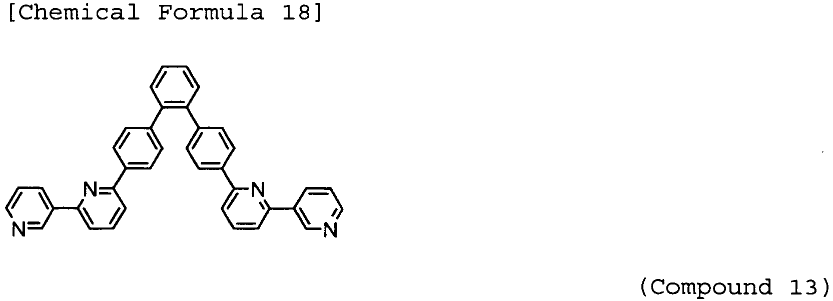

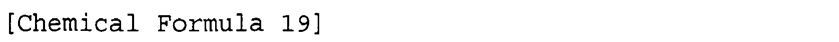

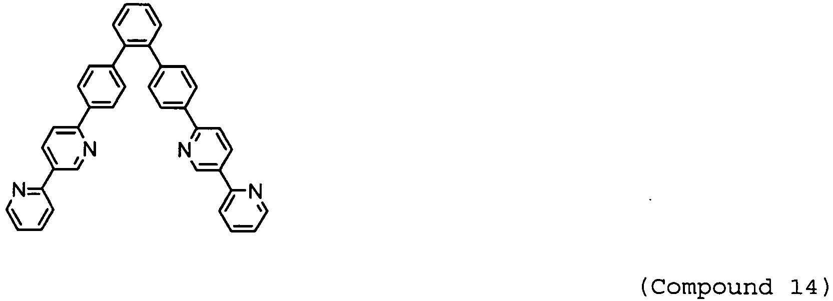

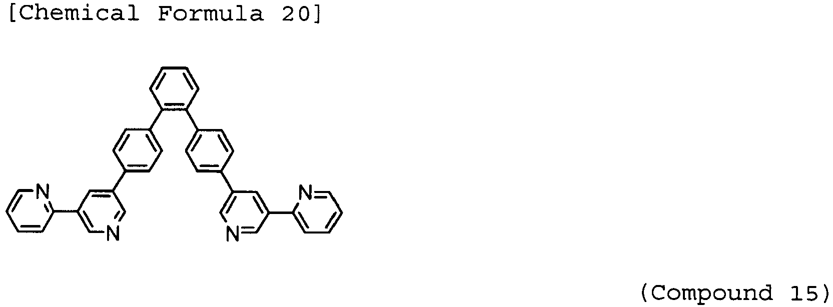

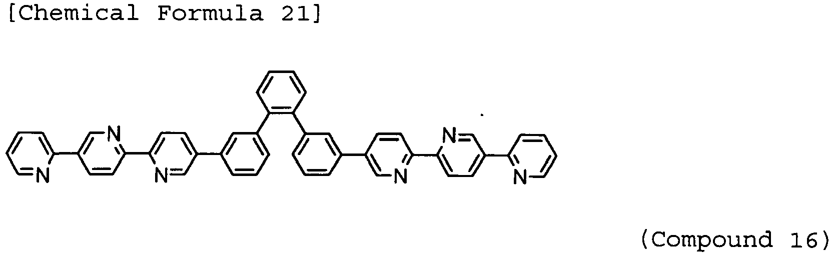

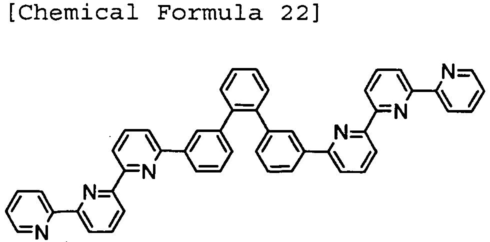

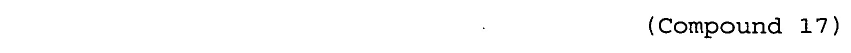

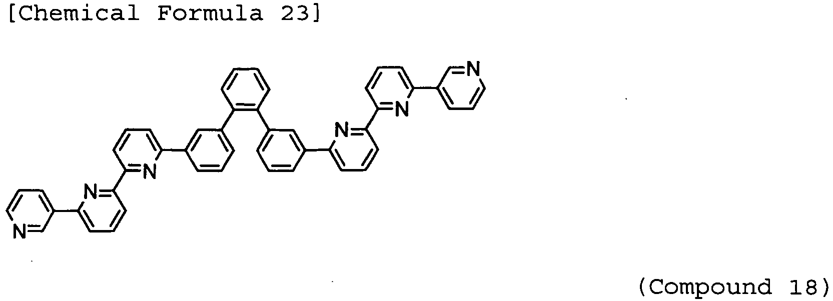

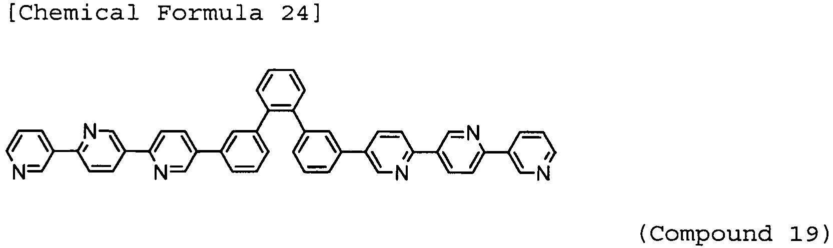

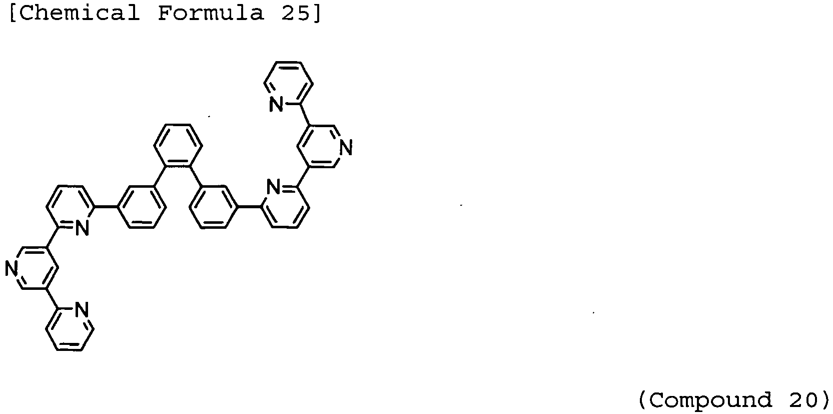

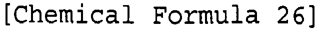

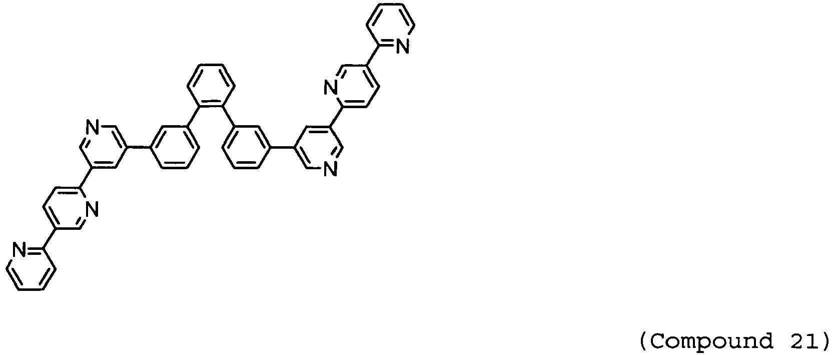

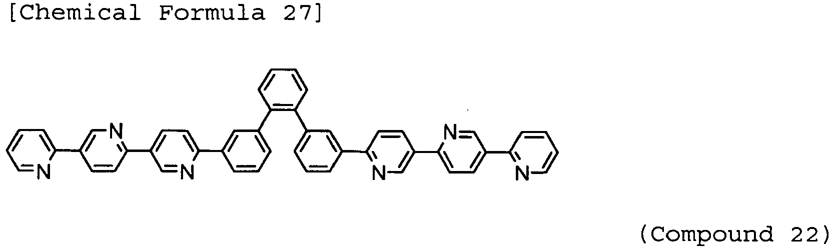

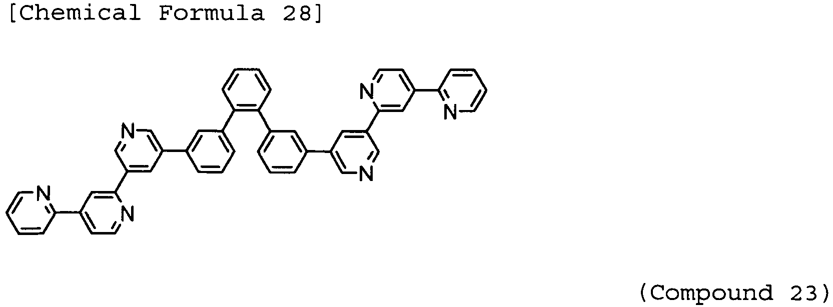

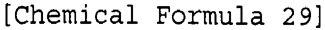

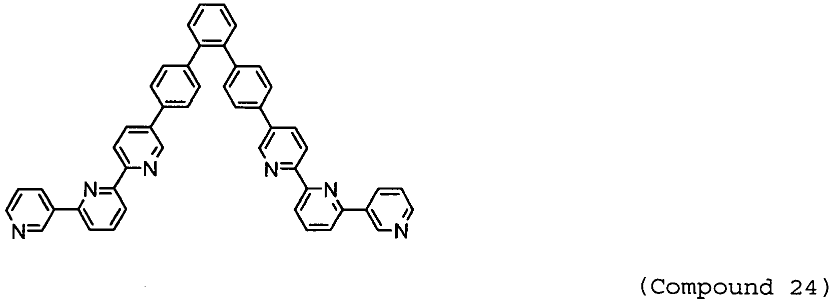

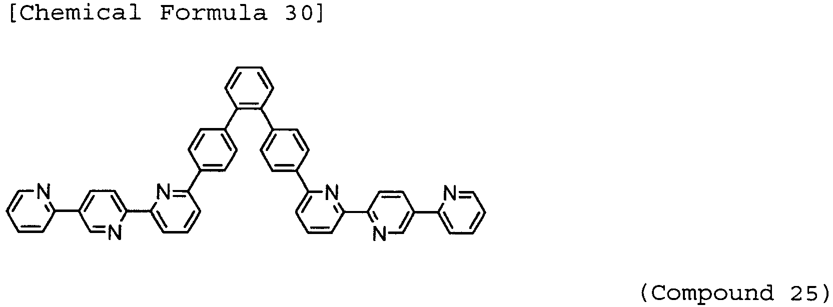

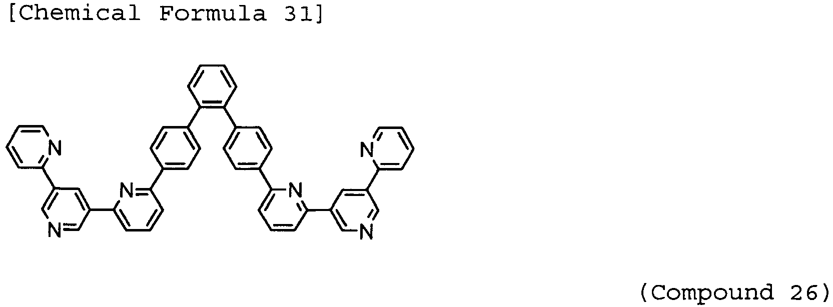

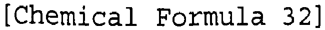

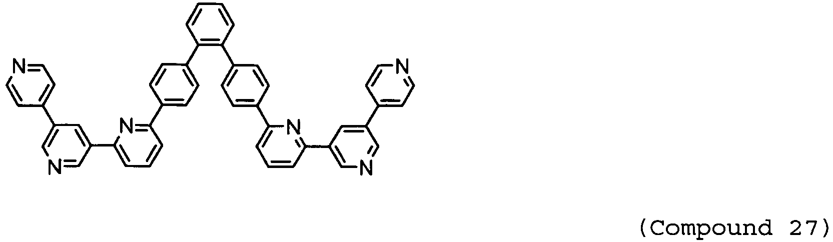

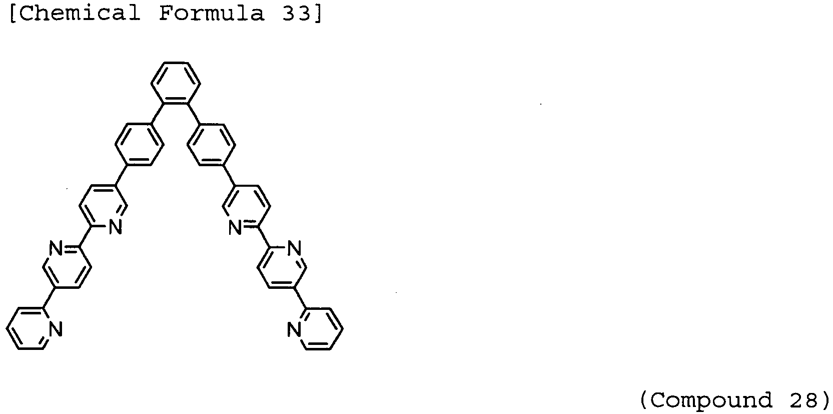

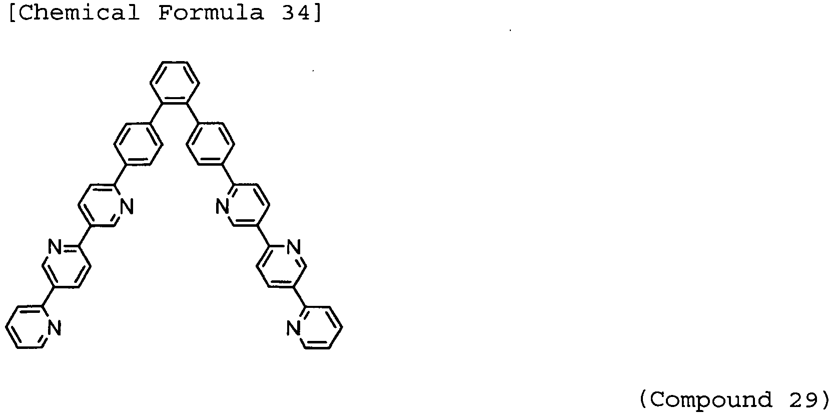

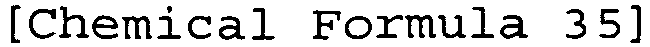

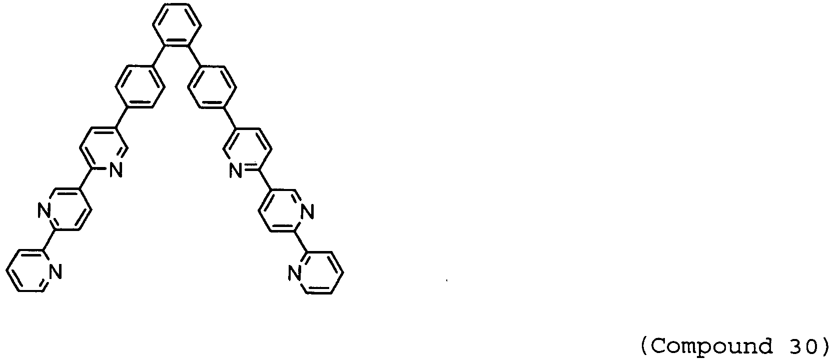

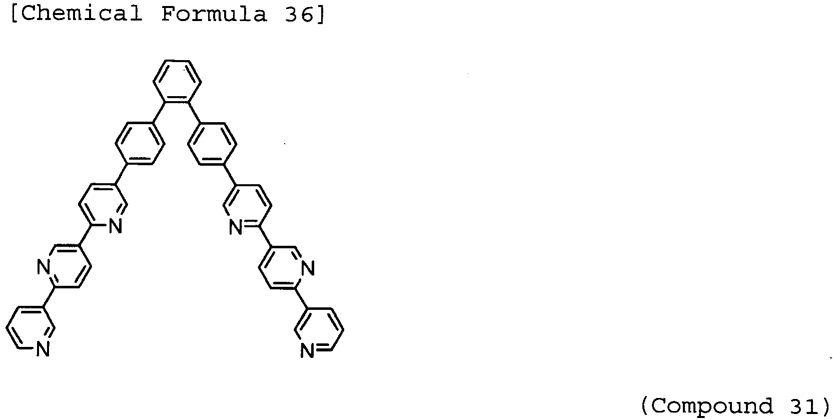

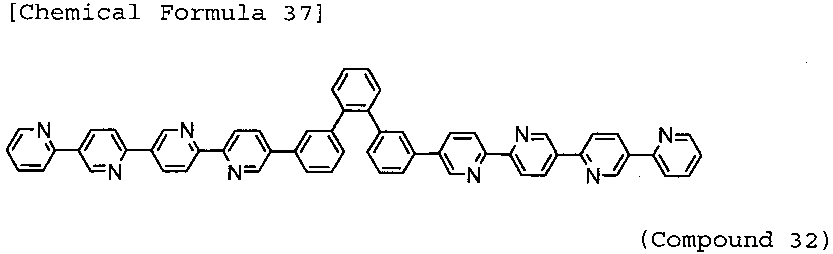

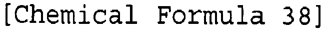

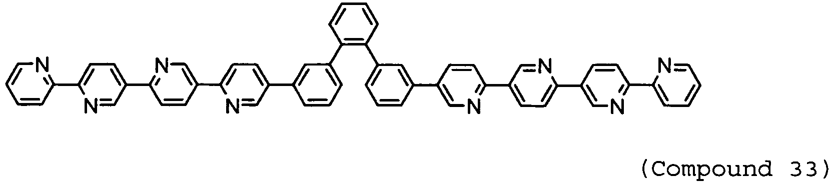

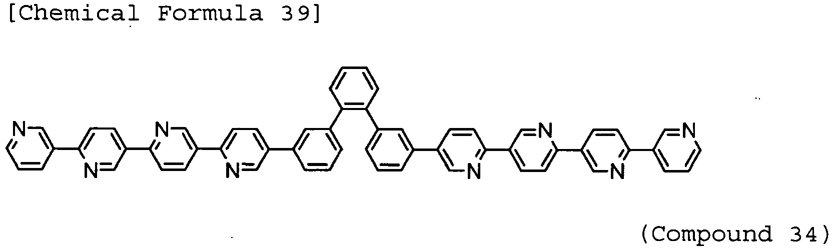

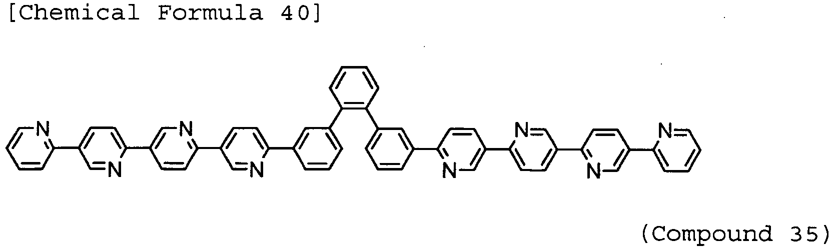

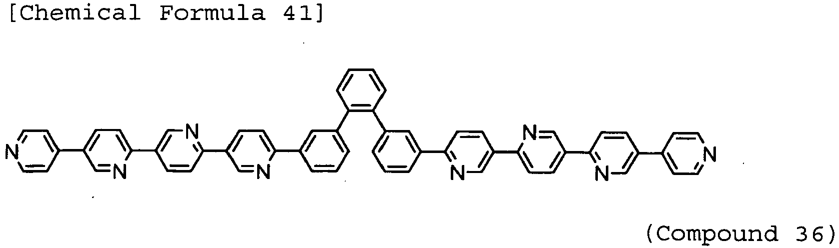

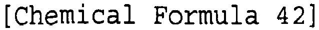

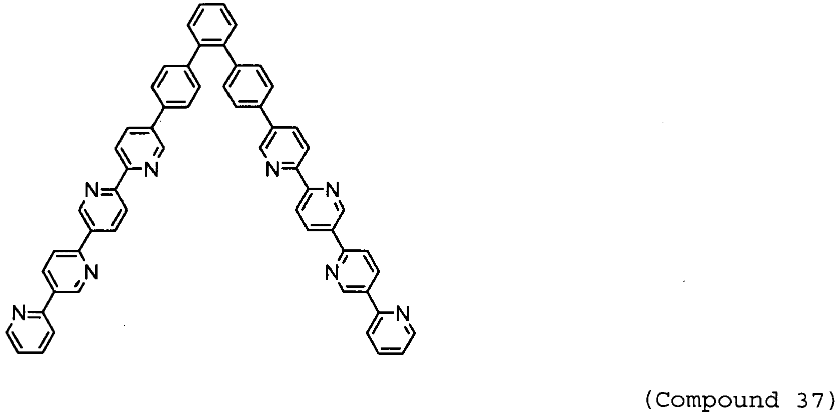

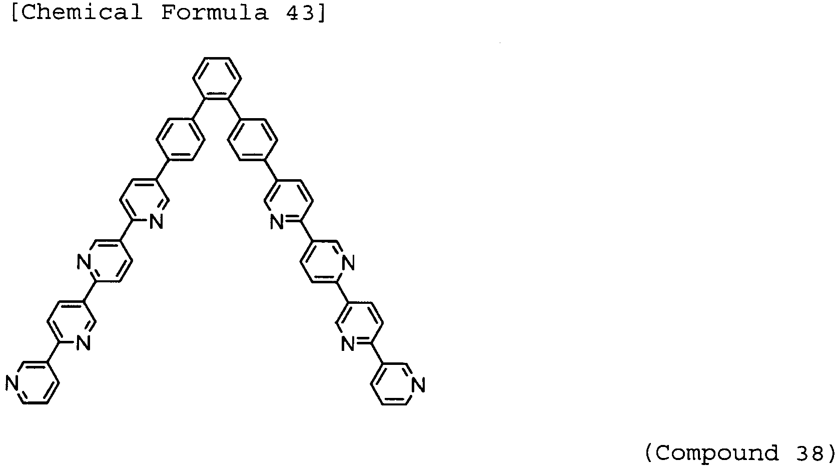

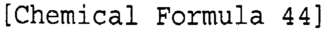

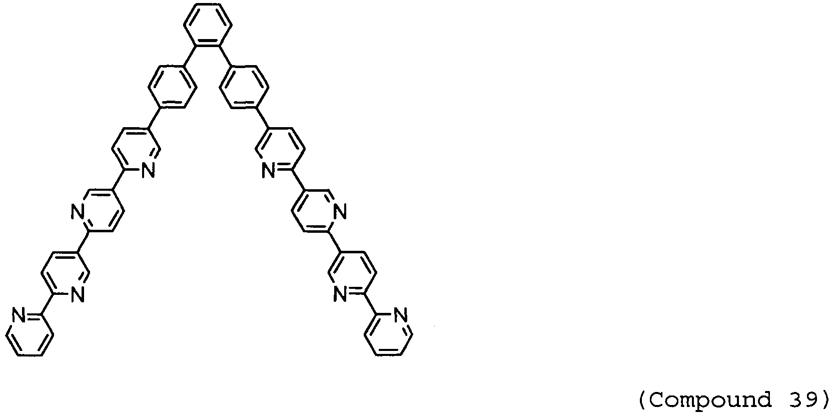

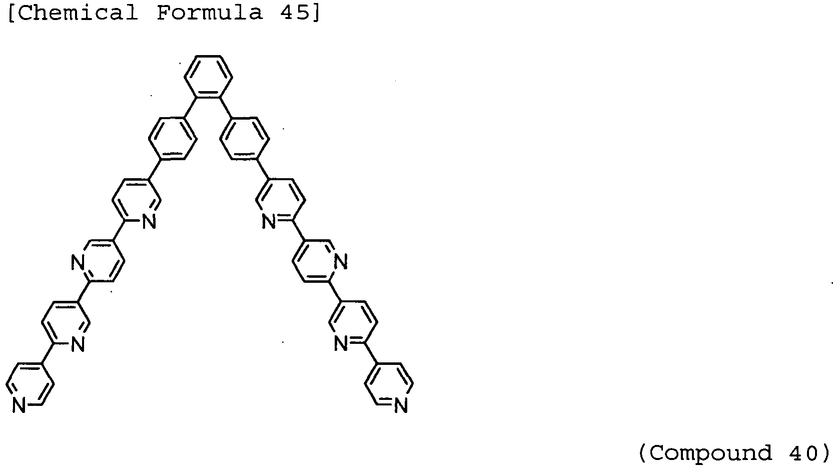

These compounds were purified by methods such as column chromatography, adsorption using, for example, a silica gel, activated carbon, or activated clay, and recrystallization or crystallization using a solvent. The compounds were identified by using methods such as NMR analysis. Melting point, glass transition point (Tg), and work function were taken for the measurement of physical properties. Melting point can be used as an index of ease of vapor deposition, glass transition point (Tg) as an index of stability in the thin-film state, and the work function as an index of energy level as a light-emitting host material.

The melting point and the glass transition point (Tg) were measured using a powder, using a high-sensitive differential scanning calorimeter DSC3100S (Bruker AXS).

For the measurement of work function, a 100 nm-thick thin film was fabricated on an ITO substrate, and an atmosphere photoelectron spectrometer (AC-3; Riken Keiki Co., Ltd.) was used.

The organic EL device of the present invention may have a structure including an anode, a hole injection layer, a hole transport layer, an electron blocking layer, a light emitting layer, a hole blocking layer, an electron transport layer, and a cathode successively formed on a substrate, optionally with an electron injection layer between the electron transport layer and the cathode. Some of the organic layers in this multilayer structure may be omitted. For example, the organic EL device may be configured to include an anode, a hole transport layer, a light emitting layer, an electron transport layer, an electron injection layer, and a cathode successively formed on a substrate, or include an anode, a hole transport layer, a light emitting layer, an electron transport layer, and a cathode successively formed on a substrate.

Each of the light emitting layer, the hole transport layer, and the electron transport layer may have a laminate structure of two or more layers.

Electrode materials with a large work function, such as ITO and gold, are used as the anode of the organic EL device of the present invention. The hole injection layer of the organic EL device of the present invention may be made of various materials, including, for example, porphyrin compounds as represented by copper phthalocyanine, naphthalenediamine derivatives, starburst-type triphenylamine derivatives, triphenylamine trimers and tetramers such as an arylamine compound of a structure in which three or more triphenylamine structures are joined to each other within the molecule via a single bond or a divalent group that does not contain a heteroatom, accepting heterocyclic compounds such as hexacyano azatriphenylene, and coating-type polymer materials. These materials may be formed into a thin film by using a vapor deposition method, or other known methods such as spin coating and an inkjet method.

Examples of the material used for the hole transport layer of the organic EL device of the present invention include benzidine derivatives [such as N,N'-diphenyl-N,N'-di (m-tolyl)-benzidine (hereinafter, referred to simply as "TPD"), N,N'-diphenyl-N,N'-di(α-naphthyl)-benzidine (hereinafter, referred to simply as "NPD"), and N,N,N',N'-tetrabiphenylylbenzidine], 1,1-bis[(di-4-tolylamino)phenyl]cyclohexane (hereinafter, referred to simply as "TAPC"), various triphenylamine trimers and tetramers, and carbazole derivatives, in addition to compounds containing an m-carbazolylphenyl group. These may be deposited alone, or may be used as a single layer deposited as a mixture with other materials, or as a laminate of individually deposited layers, a laminate of layers deposited as a mixture, or a laminate of a layer deposited alone and a layer deposited as a mixture. Examples of the material used for the hole injection/transport layer include coating-type polymer materials such as poly(3,4-ethylenedioxythiophene) (hereinafter, simply "PEDOT")/poly(styrene sulfonate) (hereinafter, simply "PSS"). These materials may be formed into a thin-film by using a vapor deposition method, or other known methods such as spin coating and an inkjet method.

Further, the hole injection layer or the hole transport layer may be one obtained by the P-doping of material such as trisbromophenylamine hexachloroantimony in the material commonly used for these layers. Further, for example, polymer compounds having a TPD structure as a part of the compound structure also may be used.

Examples of the material used for the electron blocking layer of the organic EL device of the present invention include compounds having an electron blocking effect, including, for example, carbazole derivatives such as 4,4',4"-tri(N-carbazolyl)triphenylamine (hereinafter, simply "TCTA"), 9,9-bis[4-(carbazol-9-yl)phenyl]fluorene, 1,3-bis(carbazol-9-yl)benzene (hereinafter, simply "mCP"), and 2,2-bis(4-carbazol-9-ylphenyl)adamantane (hereinafter, simply "Ad-Cz"); and compounds having a triphenylsilyl group and a triarylamine structure, as represented by 9-[4-(carbazol-9-yl)phenyl]-9-[4-(triphenylsilyl)phenyl]-9 H-fluorene. These may be deposited alone, or may be used as a single layer deposited as a mixture with other materials, or as a laminate of individually deposited layers, a laminate of a layer deposited as a mixture, or a laminate of a layer deposited alone and a layer deposited as a mixture. These materials may be formed into a thin-film by using a vapor deposition method, or other known methods such as spin coating and an inkjet method.

Examples of the material used for the light emitting layer of the organic EL device of the present invention include quinolinol derivative metal complexes such as tris(8-hydroxyquinoline)aluminum (hereinafter, referred to as simply "Alq₃"), various metal complexes, anthracene derivatives, bis(styryl)benzene derivatives, pyrene derivatives, oxazole derivatives, and polyparaphenylene vinylene derivatives. Further, the light emitting layer may be configured from a host material and a dopant material. Examples of the host material include the compounds of general formula (1) having a bipyridyl group and an ortho-terphenyl structure of the present invention, mCP, thiazole derivatives, benzimidazole derivatives, and polydialkyl fluorene derivatives. Examples of the dopant material include quinacridone, coumalin, rubrene, anthracene, perylene, derivatives thereof, benzopyran derivatives, rhodamine derivatives, and aminostyryl derivatives. These may be deposited alone, or may be used as a single layer deposited as a mixture with other materials, or as a laminate of individually deposited layers, a laminate of layers deposited as a mixture, or a laminate of a layer deposited alone and a layer deposited as a mixture.

Further, the light-emitting material may be phosphorescent light-emitting material. Phosphorescent materials as metal complexes of metals such as iridium and platinum may be used as the phosphorescent light-emitting material. Examples of the phosphorescent materials include green phosphorescent materials such as Ir(ppy)₃, blue phosphorescent materials such as FIrpic and FIr₆, and red phosphorescent materials such as Btp₂Ir(acac). Here, carbazole derivatives such as CBP, TCTA, and mCP may be used as the hole injecting and transporting host material. Compounds such as p-bis(triphenylsilyl)benzene (hereinafter, simply "UGH2"), and 2,2',2"-(1,3,5-phenylene)-tris(1-phenyl-1H-benzimidazole) (hereinafter, simply "TPBI") may be used as the electron transporting host material.

In order to avoid concentration quenching, the doping of the phosphorescent light-emitting material in the host material should preferably be made by co-evaporation in a range of 1 to 30 weight percent with respect to the whole light emitting layer.

These materials may be formed into a thin-film by using a vapor deposition method, or other known methods such as spin coating and an inkjet method.

It is also possible to produce a device of a structure that includes a light emitting layer produced with the compound of the present invention, and an adjacently laminated light emitting layer produced by using a compound of a different work function as the host material (refer to Non-Patent Document 6, for example).

The hole blocking layer of the organic EL device of the present invention may be formed by using hole blocking compounds such as various rare earth complexes, oxazole derivatives, triazole derivatives, and triazine derivatives, in addition to the compounds of general formula (1) having a bipyridyl group and an ortho-terphenyl structure of the present invention, and metal complexes of phenanthroline derivatives such as bathocuproin (hereinafter, simply "BCP"), and metal complexes of quinolinol derivatives such as aluminum(III)bis(2-methyl-8-quinolinate)-4-phenylphenolate (hereinafter, referred to simply as "BAlq"). These materials may also serve as the material of the electron transport layer. These may be deposited alone, or may be used as a single layer deposited as a mixture with other materials, or as a laminate of individually deposited layers, a laminate of layers deposited as a mixture, or a laminate of a layer deposited alone and a layer deposited as a mixture. These materials may be formed into a thin-film by using a vapor deposition method, or other known methods such as spin coating and an inkjet method.

Examples of the material used for the electron transport layer of the organic EL device of the present invention include various metal complexes, triazole derivatives, triazine derivatives, oxadiazole derivatives, thiadiazole derivatives, carbodiimide derivatives, quinoxaline derivatives, phenanthroline derivatives, and silole derivatives, in addition to quinolinol derivative metal complexes such as Alq₃ and BAlq. These may be deposited alone, or may be used as a single layer deposited as a mixture with other materials, or as a laminate of individually deposited layers, a laminate of layers deposited as a mixture, or a laminate of a layer deposited alone and a layer deposited as a mixture. These materials may be formed into a thin-film by using a vapor deposition method, or other known methods such as spin coating and an inkjet method.

Examples of the material used for the electron injection layer of the organic EL device of the present invention include alkali metal salts (such as lithium fluoride, and cesium fluoride), alkaline earth metal salts (such as magnesium fluoride), and metal oxides (such as aluminum oxide). However, the electron injection layer may be omitted upon preferably selecting the electron transport layer and the cathode.

The electron injection layer or the electron transport layer may be one obtained by the N-doping of metals such as cesium in the materials commonly used for these layers.

The cathode of the organic EL device of the present invention may be made of an electrode material having a low work function (such as aluminum), or an alloy of an electrode material having an even lower work function (such as a magnesium-silver alloy, a magnesium-indium alloy, or an aluminum-magnesium alloy).

The following describes an embodiment of the present invention in more detail based on Examples. The present invention, however, is not limited to the following Examples.

### Example 1

### <Synthesis of 3,3"-bis(2,2'-bipyridin-5-yl)-1,1':2',1"-terphenyl (compound 2)>

2,5-Dibromopyridine (19.5 g), 2-pyridylzinc bromide (150 ml), tetrahydrofuran (90 ml), and tetrakis(triphenylphosphine)palladium(0) (4.33 g) were added to a nitrogen-substituted reaction vessel. After being cooled, the mixture was stirred at 0°C for 2 hours, and then at room temperature for 3 hours. The reaction mixture was added to a 10% disodium dihydrogen ethylenediamine tetraacetate aqueous solution, and stirred for 6 hours. The organic layer was collected by separation after adding chloroform (300 ml) . The organic layer was dried over anhydrous magnesium sulfate, and concentrated to obtain a crude product. The crude product was purified by column chromatography (support: silica gel, eluent: toluene) to obtain a white powder of 5-bromo-2,2'-bipyridine (11.1 g; yield 63%).

Separately, 1,2-diiodobenzene (24.4 g), 3-trimethylsilylphenylboronic acid (30 g), sodium hydroxide (8.8 g), tetrakis(triphenylphosphine)palladium(0) (4.3 g), diethylene glycol dimethyl ether (160 ml), and water (40 ml) were added to a nitrogen-substituted reaction vessel. The mixture was heated, and stirred at 95°C for 15 hours. After cooling the mixture to room temperature, water (100 ml) was added, and the organic layer was collected by separation. The organic layer was washed two times with water (100 ml), dried over anhydrous magnesium sulfate, and concentrated to obtain a crude product. The crude product was purified by column chromatography (support: silica gel, eluent: n-hexane) to obtain a white powder of 3,3"-bis(trimethylsilyl)-1,1':2',1"-terphenyl (23.3 g; yield 84%).

The 3,3"-bis(trimethylsilyl)-1,1':2',1"-terphenyl (23 g), bromine (12.6 ml), and chloroform (180 ml) were added to a nitrogen-substituted reaction vessel. The mixture was cooled, and stirred at -5°C for 3 hours, and then at room temperature for 4 hours. After adding a saturated sodium sulfite aqueous solution (90 ml), the organic layer was collected by separation. The organic layer was washed two times with water (100 ml), dried over anhydrous magnesium sulfate, and concentrated to obtain a crude product. The crude product was purified by recrystallization with ethanol, and washed with methanol to obtain a white powder of 3,3"-dibromo-1,1':2',1"-terphenyl (15.4 g; yield 65%).

The 3,3"-dibromo-1,1':2',1"-terphenyl (5.0 g), bis(pinacolato)diboron (6.9 g), potassium acetate (3.8 g), 1,4-dioxane (50 ml) predried with a 4A molecular sieve, a [1,1'-bis(diphenylphosphino)ferrocene]palladium(II)dichlor ide-dichloromethane complex (1:1; 0.3 g) were added to a nitrogen-substituted reaction vessel. The mixture was heated, and stirred at 80°C for 11 hours. Chloroform (100 ml) was added after cooling the mixture to 50°C, and the mixture was stirred for 30 minutes. The insoluble matter was removed by filtration, and the filtrate was concentrated to obtain a crude product. The crude product was purified by column chromatography [support: silica gel, eluent: ethyl acetate/n-hexane = 1/20 (v/v) to obtain a white powder of 3,3"-bis(4,4,5,5-tetramethyl-[1,3,2]dioxabororan-2-yl)-1, 1':2',1"-terphenyl (3.8 g; yield 61%).

The 3,3"-bis(4,4,5,5-tetramethyl-[1,3,2]dioxabororan-2-yl)-1, 1':2',1"-terphenyl (1.8 g), the 5-bromo-2,2'-bipyridine (1.8 g), a 2 M potassium carbonate aqueous solution (5.8 ml), tetrakis(triphenylphosphine)palladium(0) (0.2 g), toluene (40 ml), and ethanol (10 ml) were added to a nitrogen-substituted reaction vessel. The mixture was heated, and refluxed for 20 hours while being stirred. After cooling the mixture to room temperature, water (30 ml) and chloroform (100 ml) were added, and the organic layer was collected by separation. The organic layer was washed with water (100 ml), dried over anhydrous magnesium sulfate, and concentrated to obtain a crude product. The crude product was purified by column chromatography [support: NH silica gel, eluent: ethyl acetate/n-hexane = 1/5 (v/v)] to obtain a white powder of 3,3"-bis(2,2'-bipyridin-5-yl)-1,1':2',1"-terphenyl (compound 2; 1.5 g; yield 80%).

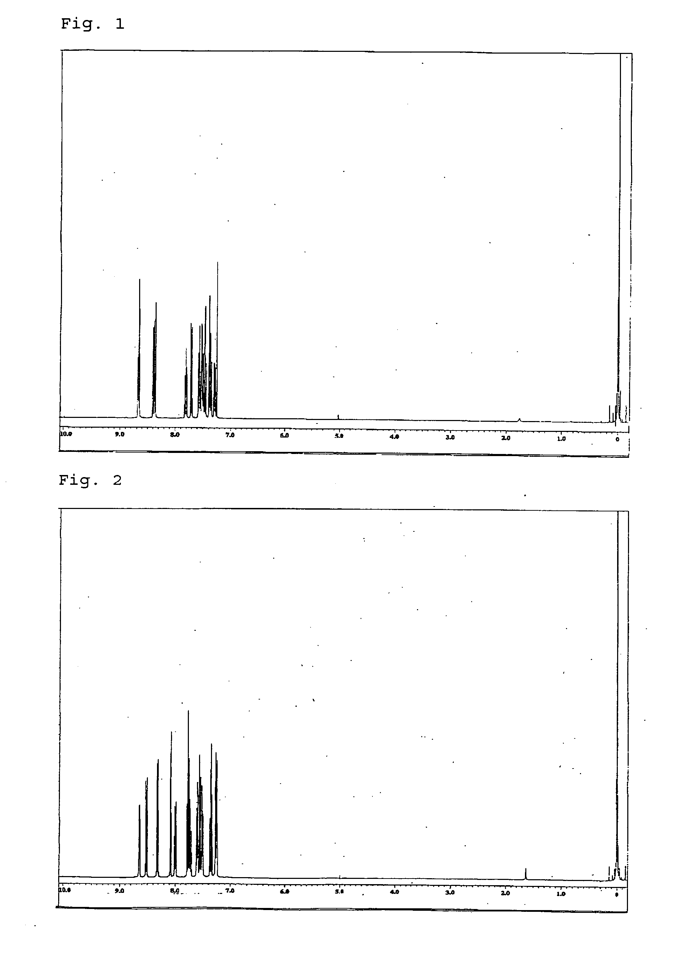

The structure of the product white powder was identified by NMR. The ¹H-NMR measurement result is shown in FIG. 1.

¹H-NMR (CDCl₃) detected 26 hydrogen signals, as follows. δ (ppm) = 8.65-8.66(2H), 8.51-8.53(2H), 8.31-8.33(2H), 8.08(2H), 7.99-8.01(2H), 7.71-7.79(4H), 7.59-7.61(2H), 7.54-7.56(2H), 7.50-7.52(2H), 7.34-7.38(2H), 7.25-7.28(4H).

### Example 2

### <Synthesis of 3,3"-bis(2,2'-bipyridin-6-yl)-1,1':2',1"-terphenyl (compound 3)>

2,6-Dibromopyridine (19.5 g), 2-pyridylzinc bromide (150 ml), tetrahydrofuran (90 ml), tetrakis(triphenylphosphine)palladium(0) (4.33 g) were added to a nitrogen-substituted reaction vessel. The mixture was cooled, and stirred at 0°C for 2 hours, and then at room temperature for 3 hours. The reaction mixture was then added to a 10% disodium dihydrogen ethylenediamine tetraacetate aqueous solution, and stirred for 6 hours. The organic layer was collected by separation after adding chloroform (300 ml) . The organic layer was dried over anhydrous magnesium sulfate, and concentrated to obtain a crude product. The crude product was purified by column chromatography (support: silica gel, eluent: toluene) to obtain a white powder of 6-bromo-2,2'-bipyridine (11.1 g; yield 63%).

The 6-bromo-2,2'-bipyridine (1.8 g), the 3,3"-bis(4,4,5,5-tetramethyl-[1,3,2]dioxabororan-2-yl)-1, 1':2',1"-terphenyl (1.8 g) synthesized in Example 1, a 2 M potassium carbonate aqueous solution (5.8 ml), tetrakis(triphenylphosphine)palladium(0) (0.2 g), toluene (40 ml), and ethanol (10 ml) were added to a nitrogen-substituted reaction vessel. The mixture was heated, and refluxed for 8 hours while being stirred. After cooling the mixture to room temperature, water (30 ml) and toluene (40 ml) were added, and the organic layer was collected by separation. The organic layer was washed with water (30 ml), dried over anhydrous magnesium sulfate, and concentrated to obtain a crude product. The crude product was purified by column chromatography [support: NH silica gel, eluent: ethyl acetate/n-hexane = 1/5 (v/v)] to obtain a white powder of 3,3"-bis(2,2'-bipyridin-6-yl)-1,1':2',1"-terphenyl (compound 3; 1.5 g; yield 75%).

The structure of the product white powder was identified by NMR. The ¹H-NMR measurement result is shown in FIG. 2.

¹H-NMR(CDCl₃) detected 26 hydrogen signals, as follows. δ (ppm) = 8.66-8.68(4H), 8.35-8.40(4H), 7.79-7.83(2H), 7.69-7.72(2H), 7.45-7.58(8H), 7.35-7.39(4H), 7.28-7.31(2H).

### Example 3

### <Synthesis of 4,4"-bis(2,2'-bipyridin-5-yl)-1,1':2',1"-terphenyl (compound 10)>

1,2-Diiodobenzene (20 g), 4-trimethylsilylphenylboronic acid (25 g), sodium hydroxide (7.4 g), tetrakis(triphenylphosphine)palladium(0) (3.6 g), diethylene glycol dimethyl ether (240 ml), and water (60 ml) were added to a nitrogen-substituted reaction vessel. The mixture was heated, and stirred at 95°C for 15 hours. After cooling the mixture to room temperature, water (100 ml) was added, and the organic layer was collected by separation. The organic layer was washed two times with water (100 ml), dried over anhydrous magnesium sulfate, and concentrated to obtain a crude product. The crude product was purified by column chromatography (support: silica gel, eluent: n-hexane) to obtain a white powder of 4,4"-bis(trimethylsilyl)-1,1':2',1"-terphenyl (21.1 g; yield 93%).

The 4,4"-bis(trimethylsilyl)-1,1':2',1"-terphenyl (21 g), bromine (11.5 ml), and chloroform (150 ml) were added to a nitrogen-substituted reaction vessel. The mixture was cooled, and stirred at -5°C for 3 hours, and then at room temperature for 4 hours. The organic layer was collected by separation after adding a saturated sodium sulfite aqueous solution (90 ml). The organic layer was then washed two times with water (100 ml), dried over anhydrous magnesium sulfate, and concentrated to obtain a crude product. The crude product was purified by recrystallization with ethanol, and washed with methanol to obtain a white powder of 4,4"-dibromo-1,1':2',1"-terphenyl (14.9 g; yield 68%).

The 4,4"-dibromo-1,1':2',1"-terphenyl (5.0 g), bis(pinacolato)diboron (7.2 g), potassium acetate (3.8 g), 1,4-dioxane (50 ml) predried with a 4A molecular sieve, and a [1,1'-bis(diphenylphosphino)ferrocene]palladium(II)dichlor ide-dichloromethane complex (1:1; 0.3 g) were added to a nitrogen-substituted reaction vessel. The mixture was heated, and stirred at 80°C for 10 hours. Chloroform (150 ml) was added after cooling the mixture to 50°C, and the mixture was stirred for 30 minutes. The insoluble matter was removed by filtration, and the filtrate was concentrated to obtain a crude product. The crude product was purified by column chromatography [support: silica gel, eluent: ethyl acetate/n-hexane = 1/5 (v/v)] to obtain a white powder of 4,4"-bis(4,4,5,5-tetramethyl-[1,3,2]dioxabororan-2-yl)-1, 1':2',1"-terphenyl (3.5 g; yield 56%).

The 4,4"-bis(4,4,5,5-tetramethyl-[1,3,2]dioxabororan-2-yl)-1, 1':2',1"-terphenyl (3.0 g), the 5-bromo-2,2'-bipyridine (3.1 g) synthesized in Example 1, a 2 M potassium carbonate aqueous solution (9.3 ml), tetrakis(triphenylphosphine)palladium(0) (0.4 g), toluene (32 ml), and ethanol (8 ml) were added to a nitrogen-substituted reaction vessel. The mixture was heated, and refluxed for 9 hours while being stirred. Water (100 ml) was added after cooling the mixture to room temperature, and the organic layer was collected by separation. The organic layer was washed two times with water (100 ml), dried over anhydrous magnesium sulfate, and concentrated to obtain a crude product. The crude product was purified by column chromatography (support: silica gel, eluent: chloroform), and recrystallized with toluene to obtain a white powder of 4,4"-bis(2,2'-bipyridin-5-yl)-1,1':2',1"-terphenyl (compound 10; 1.6 g; yield 74%).

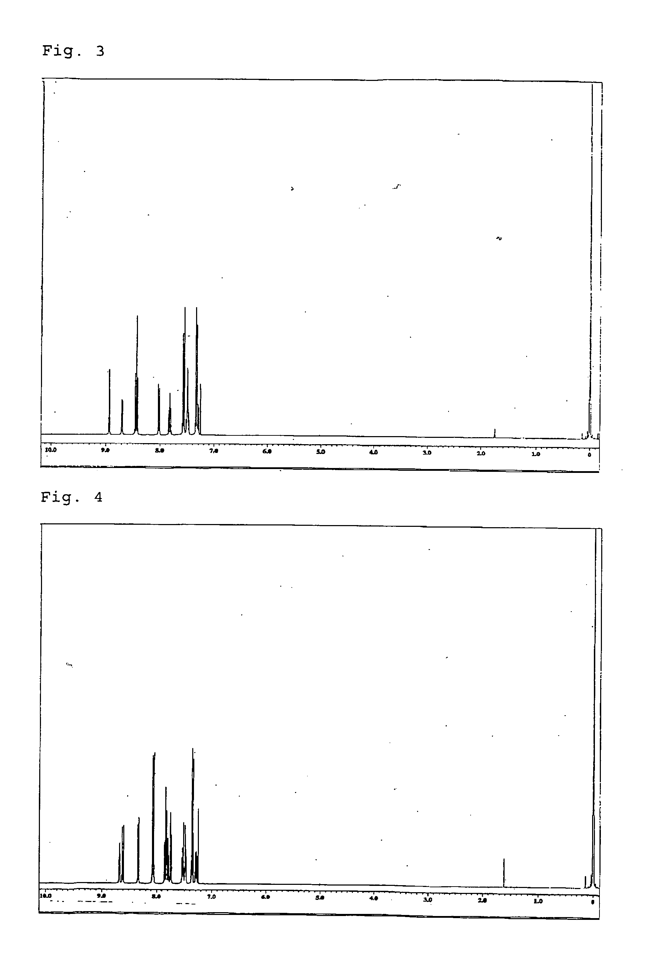

The structure of the product white powder was identified by NMR. The ¹H-NMR measurement result is shown in FIG. 3.

¹H-NMR (CDCl₃) detected 26 hydrogen signals, as follows. δ (ppm) = 8.93(2H), 8.69(2H), 8.43(4H), 8.02(2H), 7.81(2H), 7.57(4H), 7.50(4H), 7.26-7.35(6H).

### Example 4

### <Synthesis of 4,4"-bis(2,2'-bipyridin-6-yl)-1,1':2',1"-terphenyl (compound 11)>

The 4,4"-bis(4,4,5,5-tetramethyl-[1,3,2]dioxabororan-2-yl)-1, 1':2',1"-terphenyl (2.0 g) synthesized in Example 3, the 6-bromo-2,2'-bipyridine (2.0 g) synthesized in Example 2, a 2 M potassium carbonate aqueous solution (6.0 ml), tetrakis(triphenylphosphine)palladium(0) (0.2 g), toluene (32 ml), and ethanol (8 ml) were added to a nitrogen-substituted reaction vessel. The mixture was heated, and refluxed for 9 hours while being stirred. Water (100 ml) was added after cooling the mixture to room temperature, and the organic layer was collected by separation. The organic layer was washed two times with water (100 ml), dried over anhydrous magnesium sulfate, and concentrated to obtain a crude product. The crude product was purified by column chromatography (support: silica gel, eluent: chloroform), and recrystallized with toluene to obtain a white powder of 4,4"-bis(2,2'-bipyridin-6-yl)-1,1':2',1"-terphenyl (compound 11; 1.6 g; yield 74%).

The structure of the product white powder was identified by NMR. The ¹H-NMR measurement result is shown in FIG. 4.

¹H-NMR (CDCl₃) detected 26 hydrogen signals, as follows. δ (ppm) = 8.67 (2H), 8.60 (2H), 8.33 (2H), 8.06 (4H), 7.78-7.86 (4H), 7.74(2H), 7.53-7.48 (4H), 7.36(4H), 7.30-7.28 (2H).

### Example 5

The melting points and the glass transition points of the compounds of the present invention were determined using a high-sensitive differential scanning calorimeter (DSC 3100S; Bruker AXS).

| | Melting point | Glass transition point |
|---|---|---|
| Compound of Example 1 of the present invention | 182°C | 73°C |
| Compound of Example 2 of the present invention | 203°C | 72°C |

### Example 6

A 50 nm-thick vapor-deposited film was fabricated on an ITO substrate using the compounds of the present invention. The work function was measured using an atmosphere photoelectron spectrometer (Model AC-3 produced by Riken Keiki Co., Ltd.).

| | Work function |
|---|---|
| Compound of Example 1 of the present invention | 6.50 eV |
| Compound of Example 2 of the present invention | 6.49 eV |
| Compound of Example 3 of the present invention | 6.10 eV |
| Compound of Example 4 of the present invention | 6.10 eV |
| CBP | 6.00 eV |

As shown above, the compounds of the present invention have more desirable energy levels than the common light-emitting-layer host compound CBP.

### Example 7

A 50 nm-thick codeposition film was formed by the dual vapor deposition of the compound of the present invention and the green phosphorescent material Ir (ppy)₃ at a deposition rate ratio of 94 : 6 (the compound of the present invention: Ir(ppy)₃). The codeposition film was measured for fluorescence quantum yield by being irradiated with excitation light with a fluorescence quantum yield measurement device (Hamamatsu Photonics).

| | Fluorescence quantum yield |
|---|---|
| Compound of Example 1 of the present invention/Ir(ppy)₃ | 71.5% |
| Compound of Example 2 of the present invention/Ir(ppy)₃ | 67.3% |
| CBP/Ir(ppy)₃ | 79.2% |

As demonstrated above, the compounds of the present invention have the same levels of triplet energy confining capability as the common green phosphorescent host material CBP.

### Example 8

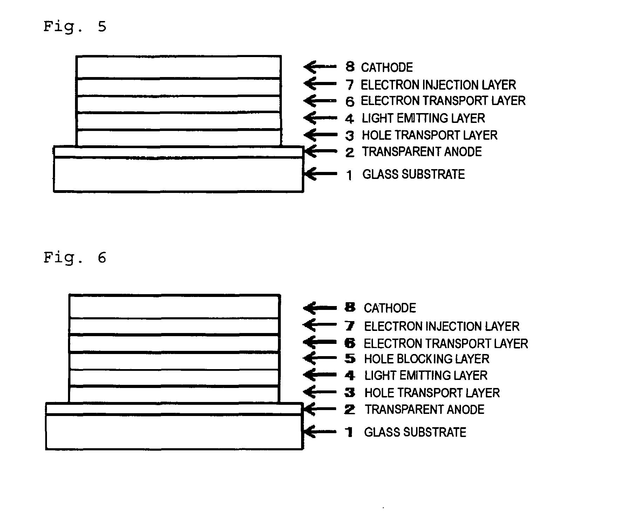

The organic EL device, as illustrated in FIG. 5, was fabricated from a hole transport layer 3, a light emitting layer 4, an electron transport layer 6, an electron injection layer 7, and a cathode (aluminum electrode) 8 successively formed by vapor deposition on a glass substrate 1 that had been provided beforehand with an ITO electrode as a transparent anode 2.

Specifically, the glass substrate 1 having ITO (thickness 100 nm) formed thereon was washed with an organic solvent, and subjected to a UV ozone treatment to wash the surface. The glass substrate with the ITO electrode was then installed in a vacuum vapor deposition apparatus, and the pressure was reduced to 0.001 Pa or less. This was followed by formation of the hole transport layer 3 by vapor depositing the compound 41 of the structural formula below over the transparent anode 2 in a thickness of 50 nm at a deposition rate of 2 Å/s. Then, the light emitting layer 4 was formed on the hole transport layer 3 in a thickness of 20 nm by the dual vapor deposition of the compound of Example 1 of the present invention (compound 2) and the green phosphorescent material Ir(ppy)₃ at a deposition rate ratio of 94:6 (compound 2:Ir(ppy)₃). The electron transport layer 6 was then formed on the light emitting layer 4 by forming Alq₃ in a thickness of 30 nm at a deposition rate of 2 Å/s. The electron injection layer 7 was then formed on the electron transport layer 6 by forming lithium fluoride in a thickness of 1 nm at a deposition rate of 0.1 Å/s. Finally, the cathode 8 was formed by vapor depositing aluminum in a thickness of 100 nm. The characteristics of the organic EL device thus fabricated were measured in an atmosphere at ordinary temperature.

Table 1 summarizes the results of the emission characteristics measurements performed by applying a DC voltage to the organic EL device fabricated with the compound of Example 1 of the present invention (compound 2).

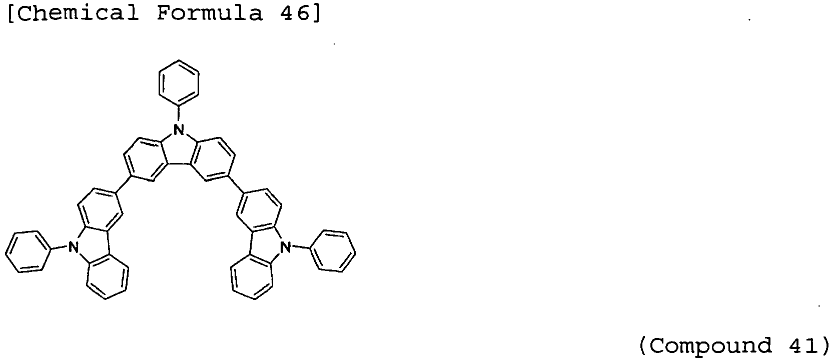

### Example 9

An organic EL device was fabricated under the same conditions used in Example 8, except that the material of the light emitting layer 4 used in Example 8 was changed to the compound of Example 2 of the present invention (compound 3). The characteristics of the organic EL device thus fabricated were measured in an atmosphere at ordinary temperature. Table 1 summarizes the results of the emission characteristics measurements performed by applying a DC voltage to the organic EL device.

### Example 10

An organic EL device was fabricated under the same conditions used in Example 8, except that the material of the light emitting layer 4 used in Example 8 was changed to the compounds of Examples 3 and 4 of the present invention (compounds 10 and 11). The characteristics of the organic EL device thus fabricated were measured in an atmosphere at ordinary temperature. The organic EL devices had desirable emission characteristics under applied DC voltage.

### Comparative Example 1

For comparison, an organic EL device was fabricated under the same conditions used in Example 8, except that the material of the light emitting layer 4 used in Example 8 was changed to CBP. The characteristics of the organic EL device thus fabricated were measured in an atmosphere at ordinary temperature. Table 1 summarizes the results of the emission characteristics measurements performed by applying a DC voltage to the organic EL device.

### Comparative Example 2

The organic EL device, as illustrated in FIG. 6, was fabricated from a hole transport layer 3, a light emitting layer 4, a hole blocking layer 5, an electron transport layer 6, an electron injection layer 7, and a cathode (aluminum electrodes) 8 successively formed by vapor deposition on a glass substrate 1 that had been provided beforehand with an ITO electrode as a transparent anode 2.

Specifically, the glass substrate 1 having ITO (thickness 100 nm) formed thereon was washed with an organic solvent, and subjected to a UV ozone treatment to wash the surface. The glass substrate with the ITO electrode was then installed in a vacuum vapor deposition apparatus, and the pressure was reduced to 0.001 Pa or less. This was followed by formation of the hole transport layer 3 by vapor depositing the compound 41 of the structural formula above over the transparent anode 2 in a thickness of 50 nm at a deposition rate of 2 Å/s. Then, the light emitting layer 4 was formed on the hole transport layer 3 in a thickness of 20 nm by the dual vapor deposition of CBP and the green phosphorescent material Ir(ppy)₃ at a deposition rate ratio of 94:6 (CBP:Ir(ppy)₃). The hole blocking layer 5 was then formed on the light emitting layer 4 by forming BCP in a thickness of 10 nm at a deposition rate of 2 Å/s. The electron transport layer 6 was then formed on the hole blocking layer 5 by forming Alq₃ in a thickness of 30 nm at a deposition rate of 2 Å/s. The electron injection layer 7 was then formed on the electron transport layer 6 by forming lithium fluoride in a thickness of 1 nm at a deposition rate of 0.1 Å/s. Finally, the cathode 8 was formed by vapor depositing aluminum in a thickness of 100 nm. The characteristics of the organic EL device thus fabricated were measured in an atmosphere at ordinary temperature.

**Table 1**

| | Light emitting layer | Hole blocking layer | Voltage [V] (@10mA/cm²) | Luminance [cd/m²] (@10mA/cm²) | External quantum efficiency (@10mA/cm²) | Power efficiency [lm/W] (@10mA/cm²) |
|---|---|---|---|---|---|---|
| Ex. 8 | Compound 2 | None | 6.7 | 4011 | 11.75 | 14.8 |
| Ex. 9 | Compound 3 | None | 8.7 | 4200 | 11.89 | 13.5 |
| Com. Ex. 1 | CBP | None | 8.3 | 1524 | 4.32 | 5.5 |
| Com. Ex. 2 | CBP | BCP | 8.9 | 4060 | 10.89 | 14.2 |

As can be seen in Table 1, the external quantum efficiency at 10 mA/cm² current density was 11.75% and 11.89% in Examples 8 and 9, respectively, higher than 5.5% of Comparative Example 1 in which CBP was used as light emitting layer material. The external quantum efficiency values in Examples 8 and 9 were also higher than the value 10.89% of Comparative Example 2 in which CBP was used as light emitting layer material, and the device additionally included the BCP hole blocking layer.

As is clear from these results, the organic EL devices using the compounds having a bipyridyl group and an ortho-terphenyl ring structure of the present invention had improved external quantum efficiency compared to the common light-emitting host material CBP. External quantum efficiency also improved over the organic EL device configured to additionally include the hole blocking layer using the common hole blocking layer material BCP.

As demonstrated above, the compounds of the present invention have desirable energy levels, and a desirable triplet energy confining capability.

### Industrial Applicability

The compounds having a bipyridyl group and an ortho-terphenyl ring structure of the present invention have desirable energy levels and a desirable triplet energy confining capability, and are thus desirable as the host compounds of a light emitting layer, and as hole blocking compounds. The organic EL device produced by using the compounds can have improved luminance and luminous efficiency over conventional organic EL devices.

### Description of Reference Numerals and Signs

- 1: Glass substrate
- 2: Transparent anode
- 3: Hole transport layer
- 4: Light emitting layer
- 5: Hole blocking layer
- 6: Electron transport layer
- 7: Electron injection layer
- 8: Cathode

## Claims

1. A compound of the following general formula (1) having a bipyridyl group and an ortho-terphenyl structure, 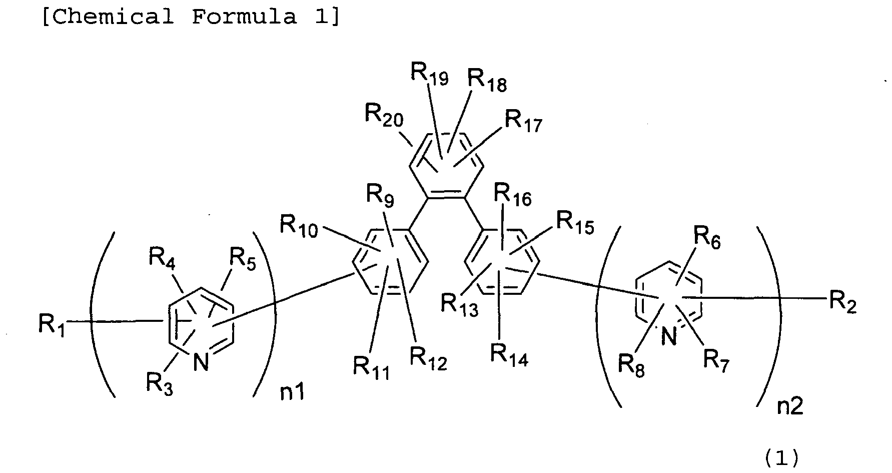 wherein R₁ to R₂₀ may be the same or different, and represent a hydrogen atom, a deuterium atom, a fluorine atom, a chlorine atom, cyano, trifluoromethyl, linear or branched alkyl of 1 to 6 carbon atoms that may have a substituent, a substituted or unsubstituted aromatic hydrocarbon group, a substituted or unsubstituted aromatic heterocyclic group, or a substituted or unsubstituted condensed polycyclic aromatic group, and wherein n1 and n2 may be the same or different, and represent 2 or 3, and the plurality of R₃ to R₈ may be the same or different.

2. The compound having a bipyridyl group and an ortho-terphenyl structure according to claim 1, wherein the compound is represented by the following general formula (1'), 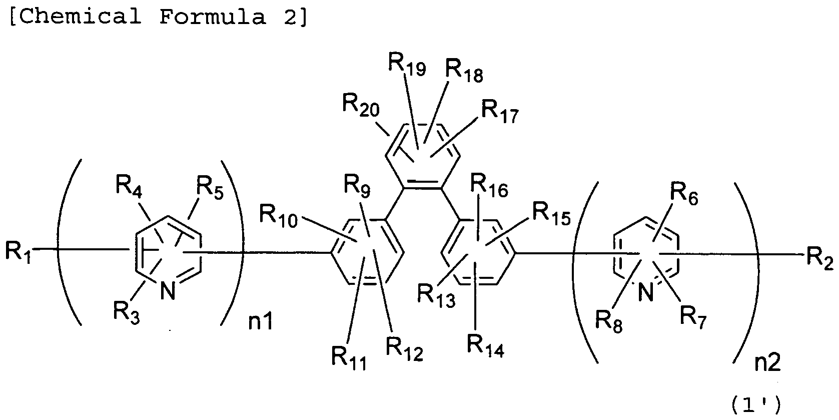 wherein R₁ to R₂₀ may be the same or different, and represent a hydrogen atom, a deuterium atom, a fluorine atom, a chlorine atom, cyano, trifluoromethyl, linear or branched alkyl of 1 to 6 carbon atoms that may have a substituent, a substituted or unsubstituted aromatic hydrocarbon group, a substituted or unsubstituted aromatic heterocyclic group, or a substituted or unsubstituted condensed polycyclic aromatic group, and wherein n1 and n2 may be the same or different, and represent 2 or 3, and the plurality of R₃ to R₈ may be the same or different.

3. The compound having a bipyridyl group and an ortho-terphenyl structure according to claim 1, wherein the compound is represented by the following general formula (1''), 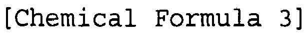 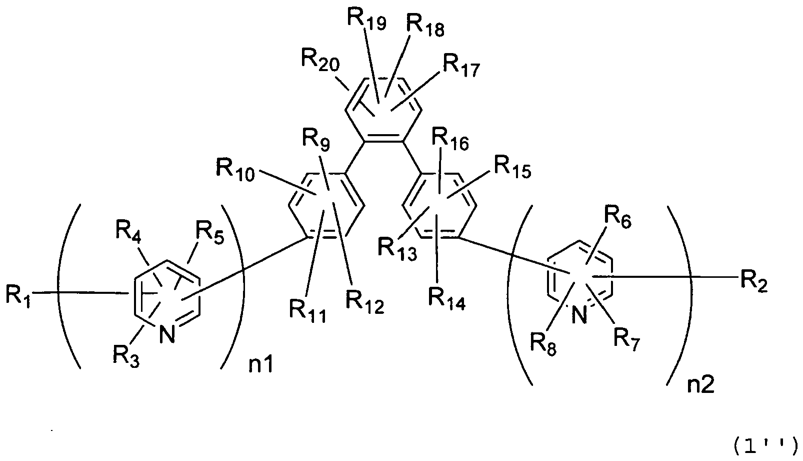 wherein R₁ to R₂₀ may be the same or different, and represent a hydrogen atom, a deuterium atom, a fluorine atom, a chlorine atom, cyano, trifluoromethyl, linear or branched alkyl of 1 to 6 carbon atoms that may have a substituent, a substituted or unsubstituted aromatic hydrocarbon group, a substituted or unsubstituted aromatic heterocyclic group, or a substituted or unsubstituted condensed polycyclic aromatic group, and wherein n1 and n2 may be the same or different, and represent 2 or 3, and the plurality of R₃ to R₈ may be the same or different.

4. An organic electroluminescent device comprising a pair of electrodes, and one or more organic layers sandwiched between the pair of electrodes, wherein a compound of the following general formula (1) having a bipyridyl group and an ortho-terphenyl structure is used as constituent material of at least one of the organic layers, 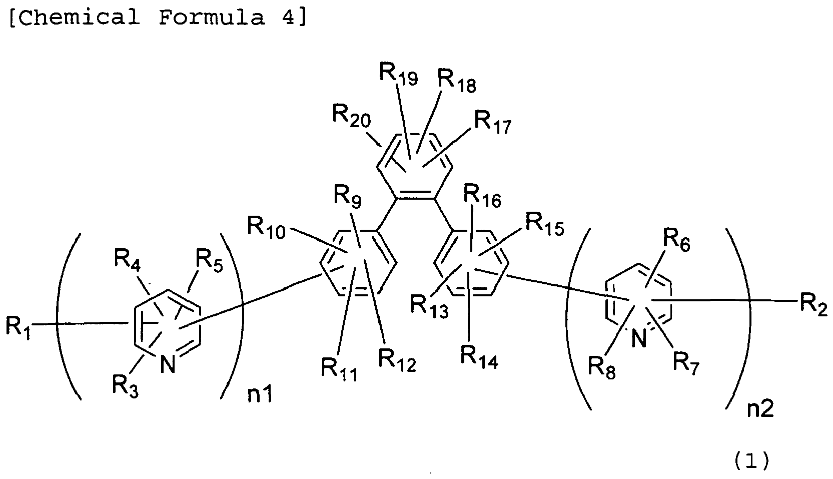 wherein R₁ to R₂₀ may be the same or different, and represent a hydrogen atom, a deuterium atom, a fluorine atom, a chlorine atom, cyano, trifluoromethyl, linear or branched alkyl of 1 to 6 carbon atoms that may have a substituent, a substituted or unsubstituted aromatic hydrocarbon group, a substituted or unsubstituted aromatic heterocyclic group, or a substituted or unsubstituted condensed polycyclic aromatic group, and wherein n1 and n2 may be the same or different, and represent 2 or 3, and the plurality of R₃ to R₈ may be the same or different.

5. The organic electroluminescent device according to claim 4, wherein the organic layer is a light emitting layer, and wherein the compound represented by the general formula (1) is used as at least one of constituent materials in the light emitting layer.

6. The organic electroluminescent device according to claim 4, wherein the organic electroluminescent device includes a pair of electrodes, and a phosphorescent light-emitting material-containing light emitting layer and one or more organic layers sandwiched between the pair of electrodes, and wherein the compound of the general formula (1) having a bipyridyl group and an ortho-terphenyl structure is used as at least one of constituent materials in the light emitting layer.

7. The organic electroluminescent device according to any one of claims 4 to 6, wherein the phosphorescent light-emitting material is a metal complex that contains iridium or platinum.
